Europäisches Patentamt

European Patent Office

Office européen des brevets

(11) Publication number: **0 166 055 B1**

# EUROPEAN PATENT SPECIFICATION

(45) Date of publication of patent specification: **18.03.92**

(51) Int. Cl.⁵: **A61K 37/18**, A61K 37/02, A61K 7/16

(21) Application number: **84309022.6**

(22) Date of filing: **21.12.84**

(54) Caries inhibition.

(30) Priority: **22.12.83 AU 2945/83**

(43) Date of publication of application:
**02.01.86 Bulletin 86/01**

(45) Publication of the grant of the patent:
**18.03.92 Bulletin 92/12**

(84) Designated Contracting States:
**AT CH DE FR GB LI NL SE**

(56) References cited:
**WO-A-82/03008**
**GB-A- 1 579 974**

**PATENT ABSTRACTS OF JAPAN, unexamined applications, field C, vol. 7, no. 252, November 9, 1983 THE PATENT OFFICE JAPANESE GOVERNMENT page 138 C 194**

**CHEMICAL ABSTRACTS, vol. 104, no. 18, May 5, 1986, Columbus, Ohio, USA K. KAMATA "Dentifrices containing collagen as cleansing agent" page 432, abstracts-no. 155 741g & JP-A-61-17 508**

(73) Proprietor: **THE UNIVERSITY OF MELBOURNE Grattan Street Parkville, Victoria 3052(AU)**

Proprietor: **VICTORIAN DAIRY INDUSTRY AUTHORITY Domville Avenue Hawthorn Victoria 3122(AU)**

(72) Inventor: **Reynolds, Eric Charles University of Melbourne Grattan Street Parkville Victoria 3052(AU)**
Inventor: **Storey, Elsdon University of Melbourne Grattan Street Parkville Victoria 3052(AU)**
Inventor: **McDougall, Wallace Arthur University of Melbourne Grattan Street Parkville Victoria 3052(AU)**

(74) Representative: **Allen, Oliver John Richard et al Lloyd Wise, Tregear & Co. Norman House 105-109 Strand London, WC2R 0AE(GB)**

Rank Xerox (UK) Business Services

## Description

This invention relates to a caries- and gingivitis-inhibiting, orally ingestible composition.

Proteins such as water-soluble caseinates, alpha$_s$-caseinate and beta-caseinate are anticariogenic, as disclosed in our earlier application WO-A-8203008.

GB-A-1570074 discloses that arginine-containing oligomeric peptides which are capable of releasing arginine in the month can be used as "pH rise factors" in dentifrices, mouthwashes and foodstuffs to provide protection against caries.

It has now been found in accordance with the present invention that when caseins are digested proteolytically, there is no loss in anticariogenic activity but the digests are readily soluble in pure water and also in acidic solutions. This ready solubility of the digests has been found to enhance their ability to interact in an anticariogenic or antigingivitis manner as compared to the undigested caseins, and accordingly the digested caseins are believed to be superior anticariogenic agents.

Thus, the present invention provides a caries-and gingivitis-inhibiting orally ingestible composition comprising a caries- and gingivitis-inhibiting amount of a casein compound and an orally ingestible carrier therefor which is characterized in that said casein compound is a water-soluble proteolytic digest of a casein.

Preferred proteases for digesting casein are trypsin, pepsin, chymotripsin and pronase. Trypsin is especially preferred.

The compositions of this invention generally contain from 0.5 to 20% by weight of the digest. Preferably the digest is present up to 10%, especially up to 5%, and more especially up to 2%, by weight.

The composition of this invention may be in the form of a foodstuff, confectionery, dentifrice, tablet lozenge or capsule or or comprise a pharmacologically acceptable vehicle, solution of suspension for topical application to the teeth or a mouthwash. Other modes of administering the digest would be acceptable if pharmacologically acceptable.

Of particular interest as compositions are chewing gum, breakfast foods, ice-cream and other frozen confectionery, confectionery, sweets and cakes as these are all known as caries problem foods.

Also of particular interest are dentifrices, mouthwashes and preparations for topical application to teeth.

Alpha$_s$-casein and other caseins are obtainable from milk.

The invention will now be further described in the Tests and Examples which follow.

## TEST 1

The purpose of this test is to determine hydroxyapatite dissolution and in this respect since tooth enamel is largely composed of hydroxyapatite it is believed that useful comparisons can be made.

Double distilled, deionised water (greater than 10 mega ohms/cm) was used throughout. Analytical reagent grade chemicals were obtained from Ajax Chemicals, Australia. Hydroxyapatite-spheroidal was purchased from BDH, and phosvitin from Sigma Chemical Co., Missouri, U.S.A. The milk proteins were fractionated by the method of Zittle and Custer (1), and their purity assessed by polyacrylamide gel electrophoresis using a modification of the method of Groves and Kiddy (2).

## Methods

## Hydroxyapatite Dissolution Rate Assay

A chromatography column (pharmacia K9/15) containing 1 g. of hydroxyapatite beads was used for the demineralisation assay. Tris 5 mM, pH 8.3 containing 50 mM NaCl and 20 mg/l neomycin was used as the column influent buffer at 20°C and at a rate of 1.000±0.003 ml./min. A protein solution (10 mg./10 ml. of influent buffer) was applied to the column and 1 ml. fractions were collected before and after protein application and assayed for total calcium, phosphate and protein. From these values a rate of dissolution (nmol calcium or phosphate per min) for each 1 ml. fraction was obtained.

## Calcium, Phosphate and Protein Assays

Inorganic phosphate was measured by the method of Itaya and Ui(3) and protein by the method of Bradford (4). The determination of calcium was by atomic absorption spectrophotometry using 1% lanthanum chloride to prevent phosphate suppression.

The following proteins were used for this study:

phosvitin,
alpha$_s$-casein,
beta-casein,
kappa-casein,
alpha-lactalbumin,
beta-lactoglobulin,
borine serum albumin, and
TD-casein

TD-casein is a tryptic digest of whole casein containing alpha$_s$-, beta- and kappa-caseins.

The effect of these individual proteins on hydroxyapatite dissolution rate is shown in Table 1.

Table 1.

| Effect of test proteins on hydroxyapatite dissolution rate | | |
|---|---|---|
| Protein | Reduction in calcium dissolution rate[a] (nmol/min) | Reduction in phosphate dissolution rate (nmol/min) |
| TD-casein | 109.2±3.2b | 67.4±5.6 |
| Phosvitin | 93.1±5.4[b] | 63.8±9.4 |
| alpha$_s$-casein | 100.1±4.1[b] | 63.5±3.3 |
| beta-casein | 94.8±11.7[b] | 64.0±19.3 |
| kappa-casein | 56.3±8.9 | 33.7±6.8 |
| alpha-lactalbumin | 2.7±1.7 | 2.9±0.6 |
| beta-lactoglobulin | 17.1±1.7 | 12.5±1.2 |
| Bovine serum albumin | 31.6±4.5 | 20.5±3.3 |

a. means±SD, n = 3
b. not significantly different P>0.5

In a trial of the above test the dissolution rate of hydroxyapatite as measured by the rate of calcium and phosphate released from the hydroxyapatite column was constant over a two hour period calcium 353.6±3.9 nmol/min, phosphate 225.4±6.8 nmol/min. The dissolution rates obtained using different hydroxapatite columns showed greater variation, calcium 354.2±23.8 nmol/min, phosphate 229.6±30.8 nmol/min, n = 11. This intercolumn variation in dissolution rate could be attributable to different column packing resulting in a different HA surface area exposed.

The results show that the four phosphoproteins gave a marked reduction in the steady-state dissolution rate with phosvitin, alpha$_s$-casein, beta-casein and TD-casein all giving essentially the same reduction in calcium and phosphate dissolution. The reduction in dissolution rate obtained with the tryptic digest of whole casein shows that the active peptides are not destroyed by the enzymatic hydrolysis.

References:

1. Zittle, C.A., Custer, J.H.: Purification and some properties of alpha$_s$-casein, J. Dairy Sci 46: 1183-1189, 1963.
2. Groves, M.L., Kiddy, G.A.: Polymorphism of gamma-casein in cow's milk. Arch. Biochem. Biophys 126: 188-193, 1968.
3. Itaya, K., Ui, M.: A new micromethod for the colorimetric determination of inorganic phosphate. Clin, Chim, Acta 14: 361-366, 1966.
4. Bradford, MM.: A rapid and sensitive method for the quantitation of microgram quantities of protein utilizing the principle of protein-dye binding. Anal. Biochem. 72:248-254, 1976.

TEST 2

This test was to determine the effect of proteins on the adsorption of the bacterium Streptococcus mutans to hydroxyapatite.

Materials and Methods

Hydroxyapatite discs were prepared by pressing 150 mg of hydroxyapatite (Bio-Gel HTP, Biorad Laboratories for 5 min under 5 tons of pressure in a KBr press. The discs were hydrated then incubated with either various protein solutions or imidazole buffer (0.05M pH 7.0, containing 0.025 M NaCl). The adherence of $^3$H-labelled S.mutans PKI cells was studied by incubating the pretreated discs with $^3$H-thymidine labelled cells ($10^9$ cells/ml) suspended in the imidazole buffer. The protein solutions used were all 5 mg/ml in imidazole buffer. The proteins and polypetides studied were TD-casein alpha$_s$-casein, beta-casein, kappa-casein, phosvitin, bovine serum albumin, histone III, histone VIII, alpha-lactalbumin, beta-lactoglobulin, poly-1-lysine and poly-1-glutamate. The caseins were prepared by selective precipitation and the other proteins were purchased from Sigma Chemical Co., Missouri, U.S.A.

Results

The effect of pretreating hydroxyapatite discs with various protein solutions on the adherence of S.mutans cells is shown In Table 2.

Table 2 Effect of protein on S. mutans adherence to hydroxyapatite.

| Proteins | Type | Number of S.mutans cells adsorbed ($\times 10^7$) |
|---|---|---|
| Control | – | 1.9±0.6[a] |
| TD-casein[b] | acidic phosphoprotein digest | 0.5±0.2 |
| alpha$_{s1}$-casein | acidic phosphoprotein | 0.5±0.3 |
| beta-casein | acidic phosphoprotein | 0.4±0.4 |
| kappa-casein | acidic phosphoprotein | 0.6±0.1 |
| phosvitin | acidic phosphoprotein | 0.5±0.1 |
| BSA | acidic protein | 0.6±0.1 |
| histone III | basic protein | 2.2±0.9 |
| histone VIII | basic protein | 2.6±0.9 |
| -lactalbumin | acidic protein | 1.0±0.4 |
| -lactoglobulin | acidic protein | 0.9±0.4 |
| poly-lysine | basic poypeptide | 3.8±1.1 |
| poly-glutamate | acidic polypeptide | 0.5±0.1 |

a mean±SD, n=8; b. TD-casein-tryptic digest of casein

Conclusion

All the acidic proteins and polypeptides (especially the phosphoproteins) caused a reduction in bacterial adherence to hydroxyapatite. However, the basic proteins and polypeptides either had no effect or

4

enhanced bacterial adherence to hydroxyapatite.

TEST 3.

This test was to determine the effect of a solution of casein and a tryptic digest of casein (TD-casein) on "caries-like" changes in enamel slabs worn intra-orally on a removable appliance. The intra-oral appliance used was a modification of that used by Ostrom and Koulourides (1976). The dacron gauze was omitted and the two enamel slabs were placed with the labial surfaces facing each other approximately 0.5mm apart in order to simulate an interproximal area. This produced a space 0.5x3x3 mm$^3$ between the two enamel slabs which allowed plaque accumulation. The appliances were worn for two weeks. Eight times a day the appliances were removed and placed in solutions at 37°c for 10 min. The left side of the appliance was placed in a solution of 2% (w/v) sucrose, 2% (w/v) glucose, 140 mM KCl, 100 mM CaCl$_2$, 20mM NaCl and pH 7.0. the right side was placed in either 2% (w/v) casein or 2% (w/v) TD-casein in a solution of 2% (w/v) sucrose, 2% (w/v) glucose, 140 mM KCl, 100 mM CaCl$_2$, 20 mM NaCl and pH 7.0. At the completion of the experiment the enamel slabs were removed sectioned and subjected to microradiography and microhardness testing. The microradiography showed that the slabs exposed to the sugar-salt solution (left-side) had sub-surface, "caries-like" lesions. However, the slabs exposed to the sugar-salt solution containing either casein or TD-casein (right side) showed no "caries-like" changes. The results were confirmed by microhardness analysis. Conclusion.

Calcium caseinate or a tryptic digest at 2% (w/v) will inhibit "caries-like" changes in enamel slabs in situ. Hence, with respect to calcium caseinate's relative insolubility it would be more expedient to use a calcium salt of a tryptic digest of casein in solid form, as the latter material is quite soluble and the active peptides remain intact on proteolytic treatment. Reference: Ostrom, C.A. and Koulourides, T.: The intraoral cariogenicity test in young subjects. Caries Res., 10: 442-452, 1976.

Example 1

Preparation of a tryptic digest of casein: An aqueous mixture containing 10 gm casein, 200 mgm trypsin (trypsin TPCK obtained from Sigma Chemical Company, Missouri, U.S.A.) and 1 litre of a 100 mM ammonium bicarbonate buffered solution having a pH of 8.53 was prepared, kept at 37°C for 2 hours and then subjected to freeze drying under vacuum to remove the water and ammonium bicarbonate to produce a tryptic digest of casein as a dry powder.
The tryptic digest of casein was readily soluble in pure water and also in acidic solutions such as exist in carbonated beverages and fruit juice.

Example 2

Carbonated Beverage: 0.5% by weight of the tryptic digest of casein of Example 1 was added to a commercially available carbonated beverage.

Example 3

Fruit Juice: 0.5% by weight of the tryptic digest of casein of Example 1 was added to a commercially available fruit juice.

Example 4

Chewing Gum: A chewing gum was made up to contain 26% gum base. 10% of the tryptic digest of casein of Example 1, 40% xylitol, 6% sorbitol, 14.5% of other polyols and 3.5% of flavours and water. All percentages were by weight.

Example 5

Flour: In a device for mixing dry substances, 1% by weight of the tryptic digest of casein was blended with flour.

Example 6

Confectionery: In the preparation of confectionery 2% by weight of tryptic-digest of casein was added to the final mixture.

Example 7

Beverage: A beverage was prepared in which 1% weight of tryptic digest of casein had been dissolved.

Example 8

Tablet: A tablet was made containing 10% by weight of tryptic digest of casein together with excipients being flavouring matter and binding material.

In preparation of a typical dentifrice within the scope of this invention, the requisite salt and salts of the selected digest are incorporated into dentifrice compositions in any suitable manner depending on whether a powder, paste or liquid preparation is to be produced. For this purpose appropriate preparations of surface-active agents, binders, flavouring materials and other excipients required to achieve the required form of dentifrice are added.

The invention is further illustrated by the following examples:

Example 9

Tooth paste: A toothpaste was prepared having the following composition:

| Tryptic digest of casein | 5.0% by weight |
|---|---|
| Gum tragacanth | 1.0% " " |
| Saccharin | 0.1% " " |
| Glycerin (B.P.) | 20.0% " " |
| Sodium lauryl sulphate | 1.0% " " |
| Methyl parahydroxy benzoate | 0.1% " " |
| Flavouring and colouring | 1.0% " " |
| Dibasic calcium phosphate | 35.0% " " |
| Water | 36.8% " " |

**Claims**

1. A caries- and gingivitis-inhibiting orally ingestible composition comprising a caries- and gingivitis-inhibiting amount of a casein compound and an orally-ingestible carrier therefor, characterized in that said casein compound is a water-soluble proteolytic digest of a casein.

2. A composition according to Claim 1, wherein the digest is formed by the use of trypsin.

3. A composition according to Claim 1 or Claim 2, wherein the casein is whole casein.

4. A composition according to any preceding claim, wherein the digest is present in amount from 0.5 to 20%, preferably up to 5%, more preferably up to 2% by weight.

5. A composition according to any preceding claim, either in the form of a dentifrice, mouthwash, tablet, lozenge or capsule or in the form of a foodstuff, confectionery or beverage.

**Revendications**

1. Composition absorbable oralement inhibant les caries et la gingivite comprenant une quantité inhibant les caries et la gingivite d'un composé de caséine et d'un véhicule absorbable oralement de ce

6

composé, caractérisé en ce que le composé de caséine est un digesté protéolytique soluble dans l'eau d'une caséine.

2. Composition selon la revendication 1, dans laquelle le digesté est formé par l'usage de la trypsine.

3. Composition selon la revendication 1 ou la revendication 2, dans laquelle la caséine est la caséine entière.

4. Composition selon l'une quelconque des revendications précédentes, dans laquelle le digesté est présent en une quantité allant de 0,5 à 20 %, de préférence jusqu'à 5 %, de façon plus préférable jusqu'à 2 %, en poids.

5. Composition selon l'une quelconque des revendications précédentes, sous forme soit d'un dentifrice, d'un bain de bouche, de tablettes, de pastilles ou de capsules, soit d'un produit alimentaire, d'un produit de confiserie ou d'une boisson.

**Patentansprüche**

1. Eine karies- und gingivitishemmende, oral einnehmbare Zusammensetzung, enthaltend eine karies- und gingivitishemmende Menge einer Kaseinverbindung und einen oral einnehmbaren Träger hierfür, dadurch gekennzeichnet, daß die Kaseinverbindung ein wasserlösliches proteolytisches Aufschlußprodukt eines Kaseins ist.

2. Zusammensetzung nach Anspruch 1, worin das Aufschlußprodukt durch Einsatz von Trypsin gebildet worden ist.

3. Zusammensetzung nach Anspruch 1 oder 2, worin das Kasein insgesamt Kasein ist.

4. Zusammensetzung nach einem der vorhergehenden Ansprüche, worin das Aufschlußprodukt in einer Menge von 0,5 bis 20 Gew.-%, vorzugsweise von bis zu 5 Gew.-%, insbesondere von bis zu 2 Gew.-%, vorliegt.

5. Zusammensetzung nach einem der vorhergehenden Ansprüche, entweder in Form eines Zahnputzmittels, eines Mundwassers, einer Tablette, einer Pastille oder einer Kapsel oder in Form eines Nahrungsmittels, Konfekts oder Getränks.